# EUROPEAN PATENT APPLICATION

(11) **EP 2 008 996 A1**
(43) Date of publication of application: **31.12.2008**
(21) Application number: 07012546.3
(22) Date of filing: 27.06.2007
(51) Int. Cl.: C07C 251/76, C07D 231/12, C07D 231/14, C07D 231/16

(54) **Process for the production of pyrazoles**

(71) Applicant: Syngenta Participations AG, 4058 Basel (CH); Syngenta Limited, Guildford Surrey GU2 7YH (GB)
(72) Inventor: The designation of the inventor has not yet been filed
(74) Representative: Hölscher, Ingo

(57) **Abstract**

The present invention relates to novel processes for the production of compounds of formula I

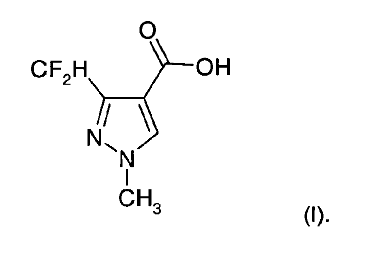

## Description

The present invention relates to novel processes for the production of 3-difluoromethyl-1-methyl-1H-pyrazole-4-carboxylic acid, which is useful as an intermediate in the production of fungicides.

Said 3-difluoromethyl-1-methyl-1H-pyrazole-4-carboxylic acid is a valuable intermediate in the production of fungicides, as described, for example, in WO 03/70705, WO 03/74491 or WO 06/35589. Fungicides are generally produced in large quantities. For example, the fungicide chlorothalonil has been produced in the year 2005 in a quantity of over 23,000 metric tons.

The aim of the present invention is therefore to provide novel processes for the production of 3-difluoromethyl-1-methyl-1H-pyrazole-4-carboxylic acid (compound of formula I) that makes it possible to prepare said acid with high regioselectivity (in respect to the two nitrogen atoms of the pyrazole ring), in high yields and good quality in an economically advantageous and easily handled way.

### First embodiment:

In a first embodiment, the present invention accordingly relates to a process for the production of a compound of formula I 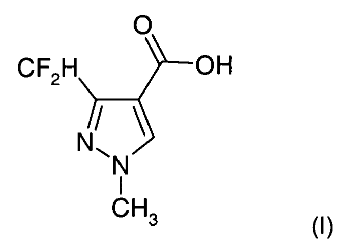 which comprises
a1) reacting a compound of formula IV (1,1-difluoroacetone) 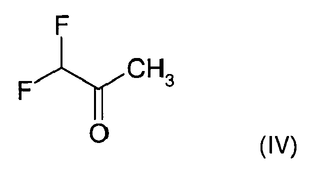 with methylhydrazine to form a compound of formula III (N-[2,2-difluoro-1-methyl-ethylidene]-N'-methyl-hydrazine) 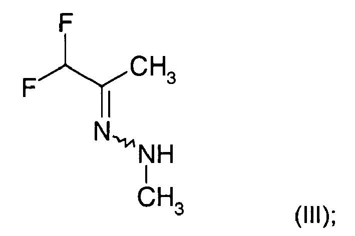
a2) converting that compound with dimethyl formamide and an activating agent into a compound of formula II (3-difluoromethyl-1-methyl-1 H-pyrazole-4-carbaldehyde) 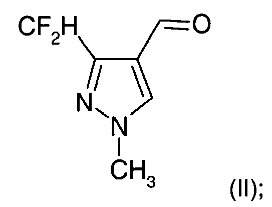 and
a3) oxidizing that compound with an oxidizing agent in the presence of a base to the compound of formula (I).

### Process step a1):

The reaction according to the invention is preferably carried out in a temperature range of from 0°C to 50°C, especially from 10°C to 25°C.

The reaction is conveniently carried out in an inert solvent. Preferred inert solvents are, for example, dimethyl formamide, xylene, toluene, mesitylene, tert-butyl benzene, chlorobenzene, 1,2-dichlorobenzene and isohexane; preferably dimethyl formamide.

In the reaction according to the invention, methylhydrazine can be used in equimolar amounts, in sub-equimolar amounts or in excess relative to compounds of formula IV, preferably methylhydrazine is used in equimolar amounts.

The reaction is carried out preferably in the presence of an acid. Suitable acids are organic acids, such as, for example, formic acid, acetic acid or propionic acid; or inorganic acids, such as, for example, hydrochloric acid or sulfuric acid. Preference is given to organic acids and special preference is given to acetic acid. Suitable amounts of acids are, for example, from 0.05 to 1 equivalents relative to compounds of formula II, especially from 0.1 to 0.5 equivalents.

The reaction time according to the invention is generally from 1 to 48 hours, preferably from 1 to 18 hours, more preferably 1 to 5 hours.

The reaction according to the invention can be carried out at normal, elevated or reduced pressure. In one embodiment of the invention the reaction is carried out at normal pressure. Typically, the compound of formula III is not isolated, but consumed in situ in process step a2).

### Process step a2):

Suitable activating agents are, for example, phosphorous oxychloride, phosgene or thionyl chloride; preferably phosphorous oxychloride.

The reaction according to the invention is preferably carried out in a temperature range of from 0°C to 130°C, especially from 75°C to 100°C.

The reaction is conveniently carried out in an inert solvent. Preferred inert solvents are the solvents used for process step a1).

In the reaction according to the invention, the activating agent, preferably phosphorous oxylchloride, is typically used in excess relative to compounds of formula III, preferably in a 2-fold to 6-fold excess.

The reaction time for the reaction according to the invention is generally from 1 to 48 hours, preferably from 1 to 24 hours, more preferably 1 to 18 hours.

The reaction according to the invention can be carried out at normal, elevated or reduced pressure. In one embodiment of the invention the reaction is carried out at normal pressure.

### Process step a3):

A suitable oxidizing agent for process step a3) is, for example, hydrogen peroxide. Suitable amounts of the oxidizing agent are, for example, at least 1 equivalent; preferably, from 10 to 20 equivalents.

Suitable bases are, for example, hydroxide bases are, for example alkali or earth alkali hydroxides, such as NaOH or KOH, with preference being given to NaOH. Suitable amounts of base are, for example, from 1 to 10 equivalents relative to compounds of formula II, especially from 2 to 5 equivalents, and very especially about 4 equivalents.

The reaction is conveniently carried out in an inert solvent. Suitable inert solvents are, for example, water; alcohols, such as methanol, ethanol, propanol or isopropanol; or aprotic solvents, such as tetrahydrofuran, *tert*-butyl methyl ether, dioxane or toluene; and mixtures thereof; water is especially preferred.

Temperatures are generally from 0°C to 120°C, with preference being given to a range from 0°C to 100°C and special preference to a range from 20°C to 60°C. In one embodiment, the temperatures are in a range from 40 to 50°C.

The reaction may be carried out at atmospheric pressure or at elevated pressure.

The reaction time for that reaction is generally from 1 to 60 hours, preferably from 1 to 6 hours.

The first embodiment of the present invention makes it possible to produce compounds of formula I in a high yield, with a high degree of regioselectivity and at low cost.

The compounds of formula II and III are valuable intermediates for the preparation of compounds of formula I and were developed specifically for the present process according to the invention. The present invention accordingly relates also to those compounds.

A further aspect of the first embodiment is a process for the production of a compound of formula II, which comprises
a1) reacting a compound of formula IV with methylhydrazine in the presence of an inert solvent to form a compound of formula III; and
a2) converting that compound with phosphorous oxychloride and dimethyl formamide into the compound of formula II.

In said aspect process steps a1) and a2) are performed as described above.

### Second embodiment:

In a second embodiment, the present invention relates to a process for the production of a compound of formula I 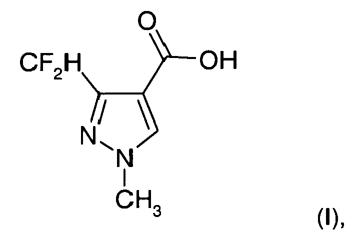 which comprises
b1) reacting a compound of formula X (dichloroacetyl chloride) 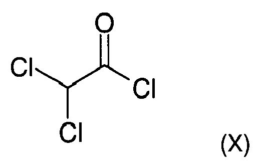 with a compound of formula Xb 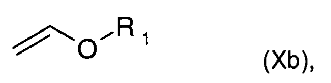 wherein R₁ is C₁-C₆alkyl, to form a compound of formula IX 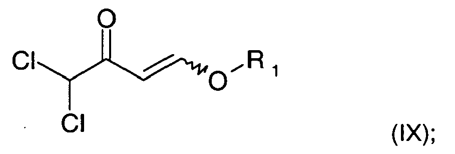 wherein R₁ is as defined under formula Xb;
b2) converting that compound with methylhydrazine into a compound of formula VIII (3-dichloromethyl-1-methyl-1 H-pyrazole) 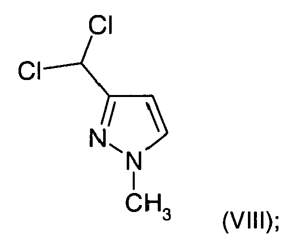
b3) fluorinating that compound with a fluorinating agent into a compound of formula VII (3-difluoromethyl-1-methyl-1 H-pyrazole) 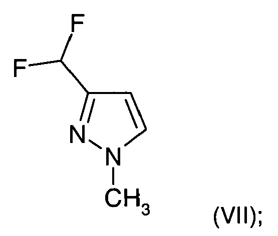
b4) brominating that compound with a brominating agent into a compound of formula VI (4-bromo-3-difluoromethyl-1-methyl-1 H-pyrazole) 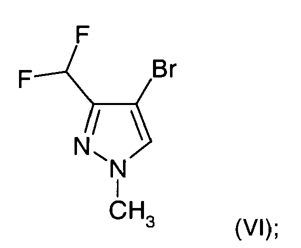
b5) converting that compound with carbon monoxide and a C₁-C₆alkanol in the presence of a palladium catalyst into a compound of formula V 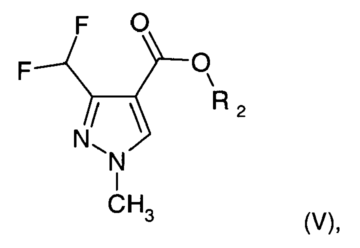 wherein R₂ is C₁-C₆alkyl; and
b6) saponifying that compound leading to the formation of the compound of formula (I) by b6.1) adding a hydroxide base or water in the presence of a base to form the anion of the compound of formula (I) and then adding an acid to form the compound of formula (I); or b6.2) adding water in the presence of an acid to form the compound of formula (I).

### Process step b1):

In process step b1) single compounds of formula Xb, such as compounds of formula Xb, wherein R₁ is ethyl, can be used or mixtures thereof. An example of such a mixture are compounds of formula Xb, wherein in R₁ is methyl, mixed with compounds of formula Xb, wherein R₁ is ethyl. In preferred compounds of formula Xb, R₁ is ethyl.

The reaction according to the invention is preferably carried out in a temperature range of from -40°C to 0°C, especially from -40°C to -20°C.

The reaction can be carried out in the presence or absence of a base. Preferred bases include pyridine, alkyl pyridines or trialkylamines.

The reaction can be carried out without solvent or in an inert solvent. Preferred inert solvents are, for example, toluene, hexane, dichloromethane or diethylether. Preferably, the reaction is carried out without a solvent.

In the reaction according to the invention, the compound of formula Xb, for example ethylvinyl ether, can be used in equimolar amounts, in sub-equimolar amounts or in excess relative to compounds of formula IV, preferably the compound of formula Xb is used in excess, more perferably in an1.5-fold to 3 -fold excess.

The reaction according to the invention may be carried out in a dry inert gas atmosphere. For example, nitrogen can be used as inert gas.

The reaction time is generally from 1 to 48 hours, preferably from 1 to 18 hours.

The reaction according to the invention can be carried out at normal, elevated or reduced pressure. In one embodiment of the invention the reaction is carried out at normal pressure.

### Process step b2):

The reaction according to the invention is preferably carried out in a temperature range of from 0°C to 50°C, especially from 10°C to 25°C.

The reaction is conveniently carried out in an inert solvent. Preferred inert solvents are, for example, xylene, toluene, mesitylene, tert-butyl benzene, chlorobenzene, 1,2-dichlorobenzene, tetrahydrofuran, diethyl ether and hexane, preferably tetrahydrofuran.

In the reaction according to the invention, methylhydrazine can be used in equimolar amounts, in sub-equimolar amounts or in excess relative to compounds of formula IX, preferably methylhydrazine is used in equimolar amounts.

The reaction time is generally from 1 to 48 hours, preferably from 1 to 18 hours, more preferably 1 to 5 hours.

The reaction according to the invention can be carried out at normal, elevated or reduced pressure. In one embodiment of the invention the reaction is carried out at normal pressure.

### Process step b3):

The fluorination according to the invention is preferably carried out in a temperature range of from 100°C to 200°C, especially from 140°C to 160°C.

The preferred fluorinating agent is tris(hydrogen fluoride)-triethylamine. The fluorinating agent is typically used in excess relative to compounds of formula VIII, preferably in a 2-fold to 5-fold excess.

The fluorination can be carried out without solvent or in an inert solvent. Preferred inert solvents are, for example, acetonitrile, chloroform, carbon tetrachloride and dichloromethane. Use of excess tris(hydrogen fluoride)-triethylamine, which then acts as solvent is preferred. The reaction time is generally from 1 to 48 hours, preferably from 1 to 18 hours, more preferably 1 to 5 hours.

The reaction according to the invention can be carried out at normal, elevated or reduced pressure. In one embodiment of the invention the reaction is carried out at elevated pressure.

### Process step b4):

The bromination according to the invention is preferably carried out in a temperature range of from 0°C to 100°C, preferably from 20°C to 40°C.

The reaction is conveniently carried out in an inert solvent. A preferred inert solvent is, for example, carbon tetrachloride.

Preferred brominating agents are, for example, bromine in the presence of an iron catalyst, N-bromosuccinimide and 1,3-dibromo-5,5-dimethylhydantoin; preferably bromine in the presence of an iron catalyst.

In case the brominating agent is bromine in the presence of an iron catalyst, said bromine is typically used in excess relative to compounds of formula VII, preferably in a 1.5-fold to 5-fold excess; and said iron catalyst is used typically in the form of iron powder and in the range of from 0.1 to 1 equivalents relative to compounds of formula VII, especially from 0.3 to 0.8 equivalents.

The reaction time is generally from 1 to 48 hours, preferably from 1 to 18 hours, more preferably 1 to 5 hours.

The reaction according to the invention can be carried out at normal, elevated or reduced pressure. In one embodiment of the invention the reaction is carried out at normal pressure.

### Process step b5):

For process step b5) the preferred C₁-C₆alkanol is ethanol; but also mixtures C₁-C₆alkanols can be used, such as a mixture of methanol/ethanol.

The reaction according to the invention is preferably carried out in a temperature range of from 50°C to 200°C, especially from 100°C to 150°C.

The reaction is conveniently carried out in a large excess of the C₁-C₆alkanol, such as a 100-fold to 150-fold excess, which then acts as solvent. Typically ethanol is used.

Typically also a large excess of carbon monoxide is used.

A suitable palladium catalyst is tris(triphenylphosphonium)palladium(II)chloride. Said palladium catalyst is used generally in the range of from 0.1 to 0.5 equivalents relative to compounds of formula VI, especially from 0.1 to 0.3 equivalents. If tris(triphenylphosphonium)palladium(II)chloride is used as palladium catalyst, then generally triphenyl phosphine is also added to the reaction mixture in amounts of from 0.5 to 0.7 equivalents relative to compounds of formula VI.

The reaction is carried out preferably in the presence of a base. Suitable bases are, for example, nitrogen-containing organic bases, such as, for example, tertiary amines, such as trialkylamines, e.g. trimethylamine, triethylamine, diisopropylethylamine (Hünig's Base), or tri-n-butylamine, N,N-dimethylaniline or N-methylmorpholine, piperidine, pyrrolidine, alkali metal or alkaline earth metal alcoholates, such as, for example, lithium, sodium or potassium alcoholates, especially methanolates, ethanolates or butanolates, or inorganic bases, such as hydroxides, e.g. NaOH or KOH, or hydrides, such as, for example, NaH. Bases to which preference is given are tertiary amines, such as trialkylamines, e.g. trimethylamine, triethylamine, diisopropylethylamine (Hünig's Base), or tri-n-butylamine, especially triethylamine. Suitable amounts of base for that reaction are, for example, from 1 to 10 equivalents, especially from 4 to 6 equivalents.

The reaction time is generally from 1 to 48 hours, preferably from 1 to 36 hours, more preferably 1 to 18 hours.

The reaction according to the invention is carried out typically at elevated pressure, such as 1 to 20 bar, preferably 10 to 15 bar.

### Process step b6):

Process step b6), the saponification of compounds of formula (V) to form the compound of formula (I), can be carried out as described under step b6.1) (alkaline saponification) or under step b6.2) (acidic saponification).

### Process step b6.1):

Step b6.1) can be divided into two sub-steps: i) the formation of the anion of the compound of formula (I) ("the anion") by adding a base and ii) the formation of the compound of formula (I) ("the free acid") by later adding an acid.

If a hydroxide base is used to form the anion, then NaOH or KOH, especially NaOH, is preferred. A suitable amount of hydroxide base is, for example, at least one equivalent relative to compounds of formula (V), preferably from 1 to 5 equivalents; more preferably from 1 to 3 equivalents.

If water in the presence of a base is used to form the anion, then inorganic bases, such as hydroxides, for example LiOH, NaOH or KOH, or carbonates, for example sodium carbonate, are preferred. In one embodiment, at least one equivalent of water and at least one equivalent of the base relative to compounds of formula (V) are used.

The formation of the anion can be carried out in an inert solvent, such as ethanol.

The formation of the anion is preferably carried out in a temperature range of from 0°C to 200°C. The reaction time for anion formation is generally from 1 to 48 hours, preferably from 1 to 18 hours. Said anion formation can be carried out at normal, elevated or reduced pressure, preferably at normal pressure.

After formation of the anion, an acid is added to form the compound of formula (I). Suitable acids are inorganic acids, such as hydrochloric acid or sulfuric acid; or organic acids, such as formic acid, acetic acid or propionic acid. Preference is given to inorganic acids and special preference is given to hydrochloric acid.

In one embodiment of the invention, the acid is added in a temperature range of from 50°C to 95°C. The reaction time for formation of the free acid is generally from 1 to 48 hours, preferably from 1 to 18 hours. Said free acid formation can be carried out at normal, elevated or reduced pressure, preferably at normal pressure.

### Process step b6.2):

In process step b6.2) the compound of formula I ("the free acid") is formed directly by acidic saponification.

The acid used in step b6.2) is typically an inorganic acid, such as hydrochloric acid or sulfuric acid; or an organic acid, such as formic acid, acetic acid or propionic acid. Preference is given to inorganic acids and special preference is given to hydrochloric acid.

Typically, aqueous solutions of the acid are used in step b6.2).

A preferable amount of water is at least one equivalent relative to compounds of formula (V). A preferable amount of acid is at least 0.01 equivalents relative to compounds of formula (V), more preferably from 0.01 to 5 equivalents; even more preferably from 1 to 5 equivalents. The formation of the free acid is preferably carried out in a temperature range of from 40°C to 100°C. The reaction time is generally from 1 to 48 hours, preferably from 1 to 18 hours. Said free acid formation can be carried out at normal, elevated or reduced pressure, preferably at normal pressure.

Also the second embodiment of the present invention makes it possible to produce compounds of formula I in a high yield, with a high degree of regioselectivity and at low cost.

The compounds of formula VI, VII and VIII are valuable intermediates for the preparation of compounds of formula I and were developed specifically for the present process according to the invention. The present invention accordingly relates also to those compounds.

Further aspects of the second embodiment are the following five individual processes:
1) A process for the production of a compound of formula IX, which comprises b1) reacting a compound of formula X with a compound of formula Xb to form the compound of formula IX.
2) A process for the production of a compound of formula VIII, which comprises b2) converting a compound of formula IX with methylhydrazine into the compound of formula VIII.
3) A process for the production of a compound of formula VII, which comprises b3) fluorinating a compound of formula VIII with a fluorinating agent into the compound of formula VII.
4) A process for the production of a compound of formula VI, which comprises b4) brominating a compound of formula VII with bromine in the presence of an iron catalyst into the compound of formula VI.
5) A process for the production of a compound of formula V, which comprises b5) converting a compound of formula VI with carbon monoxide and a C₁-C₆alkanole in the presence of a palladium catalyst into the compound of formula V.

In all five aspects above, the process steps, e.g. steps b1), b2), b3), b4) and b5) are performed as described above.

For convenience, the foregoing reactions - and further reactions, descriptions for which will follow - are summarised in scheme 1 below. In scheme 1, R₁ and R₂ stands for C₁-C₆alkyl and TREAT HF means tris(hydrogen fluoride)-triethylamine.

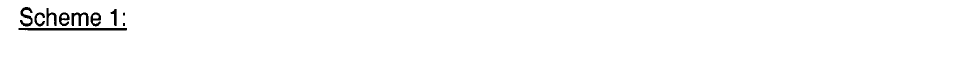

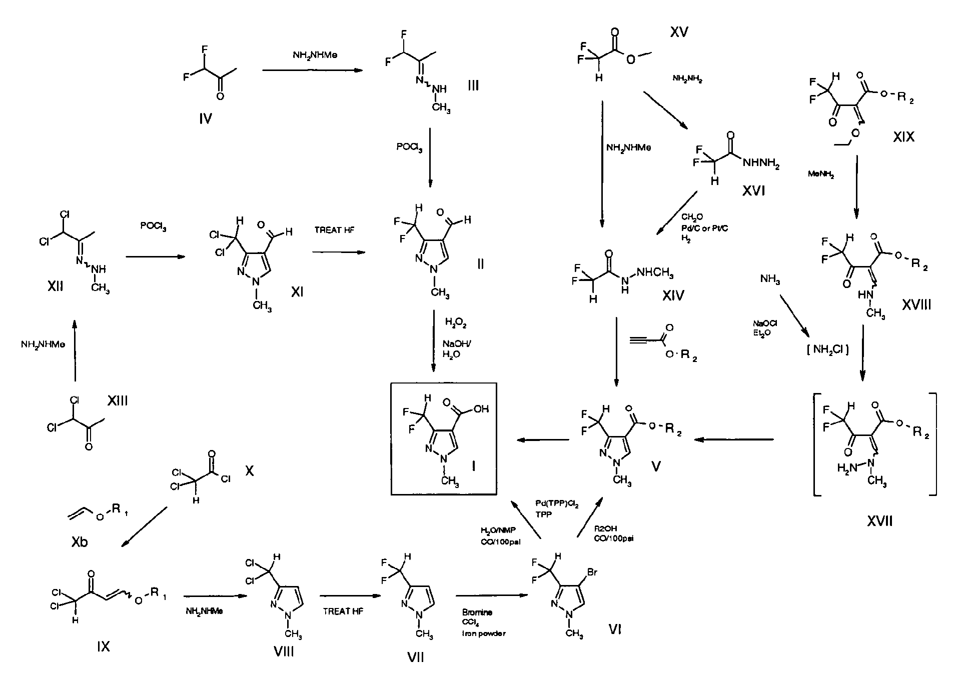

As already discussed above, the invention includes, in separate embodiments, the following multi-step processes, which involve:
(1) the formation of (I) from (IV) via (III) and (II);
(2) the formation of (II) from (IV) via (III); and
(3) the formation of (I) from (X) via (IX), (VIII), (VII), (VI) and (V).
   The invention further includes, in separate embodiments/aspects, the following processes:
(4) the formation of (IX) from (X);
(5) the formation of (VIII) from (IX);
(6) the formation of (VII) from (VIII);
(7) the formation of (VI) from (VII); and
(8) the formation of (V) from (VI).

The following non-limiting examples illustrate the invention in more detail. All following %-values are (w/w)-values unless noted otherwise.

### First embodiment of the invention:

### Example A1: Preparation of a compound of formula II from a compound of formula IV via steps a1) and a2):

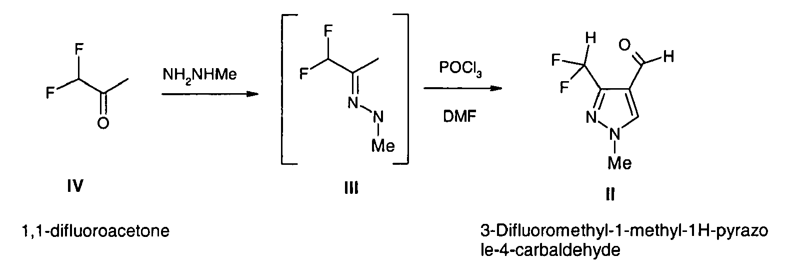

A 3-neck 500ml round bottomed flask was fitted with a magnetic stirrer, thermometer and nitrogen atmosphere. 1,1-Difluoroacetone (compound of formula IV) (10.0g), dimethylformamide (227g) and acetic acid (1.35g) were charged to the reactor. Methyl hydrazine (4.83g) was added to the agitated solution and the reaction stirred at ambient temperature overnight. This gave the desired hydrazone intermediate (compound of formula III) with >99% consumption of starting material. The reaction mass was divided into two equal parts, and one half was processed as described below: a second 3-neck 500ml round bottomed flask was fitted with a magnetic stirrer, condenser, thermometer and nitrogen atmosphere. Dimethylformamide (114g) was charged to the reactor and heated to 50°C. Phosphorous oxychloride (66.1 g) was added over 0.75hr with stirring at 45-50°C. The reaction was held at 50°C for 1 hr then cooled to 10°C. The hydrazone (compound of formula III) solution prepared above was charged over 4hr, maintaining the temperature at 5-10°C. The reaction was stirred at 80°C overnight and then cooled to ambient temperature. Dichloromethane (500ml) and ice (330g) were charged to a jacketed 3 litre reactor fitted with a mechanical agitator. The reaction mass was charged over 0.5hr to the ice/dichloromethane mixture with stirring. The pH was adjusted to 9.8 by addition of 20% sodium hydroxide solution (220ml), resulting in some precipitation of solid. Further dichloromethane (300ml) and water (750ml) were charged and the mixture filtered. The dichloromethane layer was washed with water, and the aqueous layer extracted with dichloromethane. The combined dichloromethane layers were washed with water, dried (MgSO₄) and concentrated *in vacuo* [65% yield of desired product (compound of formula II) - quantitative HPLC]. DMF removal (70°C, 3-5mbar) in a Kugelrohr distillation apparatus gave the desired product (compound of formula II) as a dark brown oil [53% yield of desired product - quantitative HPLC].
MS: 42, 51, 69, 77, 83, 112, 131, 141, 159, 160 (M⁺)
¹H NMR (CDCl₃): 4.00 (s, 3H, NCH₃), 6.88 (t, 1 H, CHF₂), 7.75 (S, 1 H, ArH), 10.0 (s, 1 H, CHO)

### Example A2: Preparation of a compound of formula I from a compound of formula 11 via step a3):

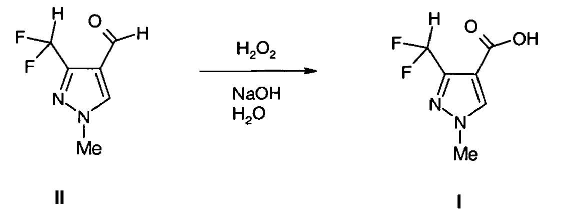

A 3-neck 250ml round bottomed flask was fitted with a magnetic stirrer and thermometer. Water (130g), the product of example 1 (compound of formula II) (6.50g) and aqueous sodium hydroxide (8.17g) were charged to the reactor, and the resulting solution heated to 40-45°C. A 35% hydrogen peroxide solution (39g) was added over 1 hr, and the mixture then stirred at 40-45°C for 0.5hr [83% yield of desired product (compound of formula I) - quantitative HPLC]. The pH of the reaction mass was adjusted to 2.5 by addition of 36% aqueous hydrochloric acid solution. The resulting precipitate was isolated by filtration and washed with water. Drying (60°C, 10mbar) gave the desired product as pale yellow, free-flowing powder [69% yield of desired product - quantitative HPLC].
MS: 42, 51, 69, 80, 88, 100, 108, 128, 137, 159, 176 (M⁺)
¹H NMR (d6-acetone): 3.98 (s, 3H, NCH₃), 7.21 (t, 1 H, CHF₂), 8.25 (S, 1 H, ArH), 11.2 (broad s, 1H, CO₂H)

### Second embodiment of the invention:

### Example B1: Preparation of a compound of formula IX from a compound of formula X via step b1):

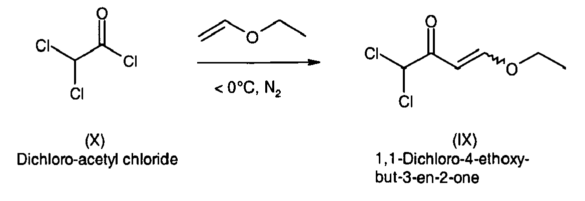

Dichloroacetyl chloride (114.9g, compound of formula X) was charged to a clean/oven dried 250ml 3-necked flask fitted with nitrogen feed, syringe feed, thermometer and a PTFE coated magnetic stirrer. The reactor was vented via a cold finger condenser and water condenser to follow. A caustic scrubber system was connected to the vent from the water condenser system.The agitation was provided by a magnetic stirrer/hotplate fitted with a drykold acetone bath. The cold finger condenser was filled with the same cooling mixture. The reactor contents were agitated and the contents cooled to <-40°C with a gentle nitrogen purge applied to the reactor. Ethylvinyl ether (113.0g) was charged to the reactor over ~8 hrs maintaining at -20 to -40°C throughout. The reaction mixture was then agitated overnight allowing it to self heat to room temperature. The following day the reaction mass was black and more viscous then before. The reaction mass was analysed by GC/GCMS and shown to have effectively completed. The reactor contents were then agitated under nitrogen whilst samples were removed for Kugelrohr distillation. A total mass of 164.4g of black liquor was obtained. The yield estimate at this stage for the product (compound of formula IX) was 48% (based on GC area% analysis) with a further potential 15% from the chlorinated intermediate present which had not dehydrochlorinated. Several distillations were carried out obtaining up to 63% recovery of the product with strengths of ~97% by GC area%. Main fraction was obtained at 137.5°C and 8mbar. The product was analysed by GC, GCMS and NMR (¹H).
GCMS: 35, 43, 48, 53, 61, 71, 76, 83, 91, 99, 109, 119, 182 (M⁺)
¹H NMR (CDCl₃): 1.40(t, 3H, CH₃CH₂O-), 4.08(q, 2H, CH₃CH₂O-), 5.83(s, 1H, CHCl2-), 6.01 (d, 1H, -CO-CH=CH), 7.80(d, 1H, -CH=CH-O-)

### Example B2: Preparation of a compound of formula VIII from a compound of formula IX via step b2):

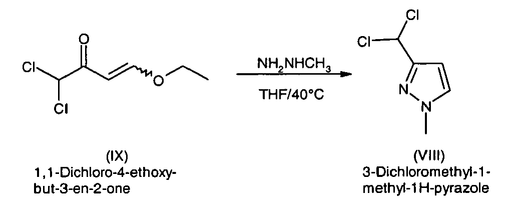

1,1-Dichloro-4-ethoxy-but-3-en-2-one (0.99g, compound of formula IX) was dissolved in tetrahydrofuran (12.5ml) in a clean/dry 50ml 3-necked flask fitted with thermometer, condenser and syringe feed system. The top of the condenser was back pressured with a gentle flow of nitrogen. The reactor contents were agitated and heated to 40°C then the N-methyl hydrazine (0.26g) was dissolved in tetrahydrofuran (25ml) and the resultant solution syringe pump fed into the reactor over a 5 hour period. The reaction mixture was agitated overnight under nitrogen to complete. The mixture was allowed to self cool over this period. A yellow/orange solution was obtained. The product solution was concentrated under vacuo and the resultant oil taken up in dichloromethane. The product solution was then washed with water, separated, dried over anhydrous magnesium sulphate and concentrated under vacuo again to give the product in form of a red oil (0.7g ~83% yield). The reaction was followed by gas chromatography throughout to assess the extent of reaction. Distillation and separation of the desired product (compound of formula VIII) from its isomer is possible - distillation with a Kugelrohr enabled separation of the isomers - the target 1, 3-isomer was predominantly obtained at 77°C and 3mbar. The unwanted 1, 5-isomer was predominantly obtained at 89°C and 4mbar. The product was analysed by GC, GCMS and NMR (¹H).
GCMS: 35, 43, 48, 53, 61, 71, 76, 83, 91, 99, 109, 119, 182 (M⁺)
¹H NMR (CDCl₃): 3.88 (s, 3H, CH₃N), 6.80 (s, 1 H, CHCl₂), 6.50 (d, 1 H, N-CH=CH-), 7.34 (d, 1 H, N-CH=CH-)
For the isomer:
¹H NMR (CDCl₃): 4.03 (s, 3H, CH₃N), 6.82 (s, 1 H, CHCl₂), 6.41 (d, 1 H, N-CH=CH-), 7.39 (d, 1H, N-CH=CH-)

### Example B3: Preparation of a compound of formula VII from a compound of formula VIII via step b3):

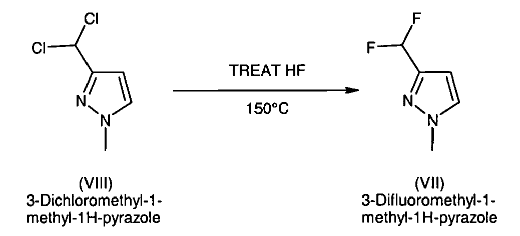

15.4 g (94 mmol) of the compound of formula VIII was charged to a clean dry Monel 100ml pressure reactor followed by 56 g (342 mmol) tris(hydrogen fluoride)-triethylamine ("TREAT HF") by syringe. The system was sealed up and agitated whilst heating the contents to 150°C. After achieving the target temperature the reaction mass was held on temperature for a further 4 hours. External cooling was then applied to cool the reactor/contents to room temperature before the reaction mass was quenched. Quenching was effected by drowning out the reactor contents (black liquid) into water (100ml). The quenched reaction mass was then extracted with methyl-t-butyl ether (3 x 25ml). After separating the organic phases were washed with brine and the organic layer was dried with magnesium sulphate, filtered and concentrated under vacuo to give the compound of formula VII in the form of a yellow/orange oil (6.3gm, ~51% yield on GC area%). The product was analysed by GC, GCMS and NMR (¹H, ¹³C and ¹⁹F).
GCMS: 38, 42, 51, 81, 113, 117, 132 (M⁺)

For the desired product: ¹H NMR (CDCl₃): 3.90 1 (s, 3H, CH₃N), 6.44 (d, 1H, N-CH=CH), 6.68 (t, 1 H, CHF₂), 7.37 (d, 1 H, N-CH=CH); ¹⁹F NMR (CDCl₃): -111.72 (d, CHF₂)
For the isomer: ¹H NMR (CDCl₃): 3.97 1 (s, 3H, CH₃N), 6.45 (d, 1H, N-CH=CH), 6.74 (t, 1H, CHF₂), 7.44 (d, 1H, N-CH=CH); ¹⁹F NMR (CDCl₃): -113.11 (d, CHF₂)

### Example B4: Preparation of a compound of formula VI from a compound of formula VII via step b4):

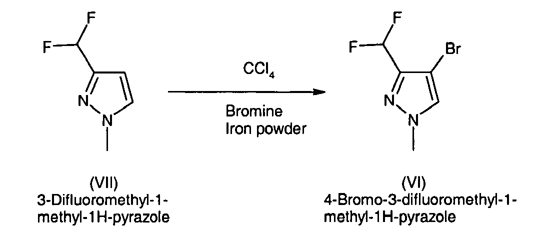

1.7 g (30 mmol) iron powder was added to 6.1 g (57 mmol) of the compound of formula VII in a 250ml 3-necked round bottom flask fitted with condenser (which was vented to a caustic scrubber system), thermometer and syringe pump feed. The first charge of carbon tetrachloride (25ml) was made to the flask and the flask contents agitated. Bromine (6.8g) was dissolved in furhter carbon tetrachloride (35ml). Coolant was applied to the condenser, and the bromine solution was fed to the reactor over 1 hr. During the addition the temperature of the reaction rose to ~29°C. GC analysis showed that the reaction was incomplete. A second charge of bromine (8.6g) in carbon tetrachloride (25ml) was then made to the reactor over a further 1 hr and the reaction mass was allowed to stand overnight. GC analysis showed that the reaction wasn't complete but additional impurities were forming at significant levels. The reaction mass was therefore quenched by treating with sodium bisulphite solution (100ml) - a small exotherm occurred to 26°C and the system was decolourised. The two phases were separated and the organic phase was then washed with further sodium bisulphite solution (50ml). The organic phase was separated from the aqueous phase and dried over magnesium sulphate. The solvent was removed by distillation to give an orange oil (7.5gm ~77% yield on actual weight and GC area% strength). The oil was distilled on a kugelrohr short path distillation system at 50 to 96°C (9 to 6 mbar). The compound of formula VI was obtained in the form of a yellow oil over 88 to 96°C (7mbar). The main fraction from the distillation was 5.97g (80% recovery or 62% through yield). Crude and distilled products were analysed by GC, GCMS and NMR (¹H and ¹⁹F).
GCMS: 42, 51, 69, 80, 88, 104, 118, 131, 159, 191, 197, 210 (M⁺)
¹H NMR (CDCl₃): 3.91 (s, 3H, CH₃N), 6.67 (t, 1 H, CHF₂), 7.44 (s, 1 H, N-CH=C-Br) ¹⁹F NMR (CDCl₃): -114.41 (d, CHF₂)

### Example B5: Preparation of a compound of formula V from a compound of formula VI via step b5):

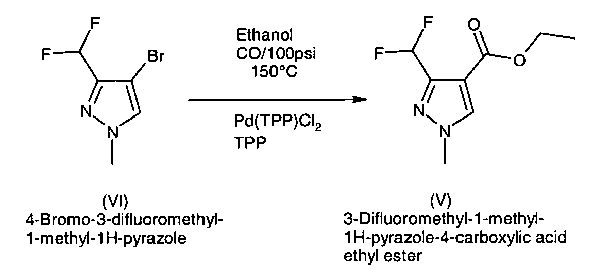

0.43 g 4-Bromo-3-difluoromethyl-1-methyl-1H-pyrazole, 10 g ethanol, 1 g triethylamine, 0.11 g tris(triphenylphosphonium)palladium(II)chloride and 0.12 g triphenyl phosphine were charged to a 50ml glass miniclave reactor fitted with a 10bar bursting disc, thermometer and gas feed. The system was pre-purged with carbon monoxide six times up to 7 bar - venting off slowly each time and agitating throughout. Finally the reactor was pressurised up to 6 bar. The system was agitated whilst heating up to 150°C and the pressure maintained at 6-7 bar by venting as necessary. When the mixture had stabilised on temperature, and pressure, it was agitated overnight under these conditions. GC analysis showed the reaction to be incomplete, so a second charge of catalyst (0.06g tris-triphenylphosphine palladium (II) chloride and 0.14g triphenylphosphine) was added to the reactor. The reaction was agitated at 150°C for a further 5hr. A third charge of catalyst was made (0.06g tris-triphenylphosphine palladium (II) chloride) and the mixture then stirred for a further for 5hr. The system was cooled, vented and the reactor contents evaporated as far as possible on a rotary evaporator. The resultant oil was quenched with an aqueous base and extracted with 3 x 25ml of ether. The combined ether extracts were washed with brine, dried over magnesium sulphate and concentrated under vacuo to yield a red/brown oil (0.5g). The aqueous base extracts were acidified and back extracted with 3 x 25ml of ether. The extracts were combined, dried over magnesium sulphate and concentrated under vacuo. Derivatised GC analysis showed that the desired 1, 3-pyrazole isomer (compound of formula V) was present in virtually all phases obtained from the work-up (HPLC analysis). Most of the product, and its isomer, were present in the ether extract from the initial base wash. The product was not isolated but compared to reference material by HPLC, GC and GCMS. Analysis via GC area% of the reaction mixture suggested a conversion of 60%.

### Example B6: Preparation of a compound of formula I from a compound of formula V via step b6):

To be delivered by Fanny.

The following illustrates other processes for the production of the compound of formula (I) as laid out in scheme 1 above (examples C1 to C8).

### Preparation of (I) from (VI):

### Example C1: Preparation of (I) from (VI):

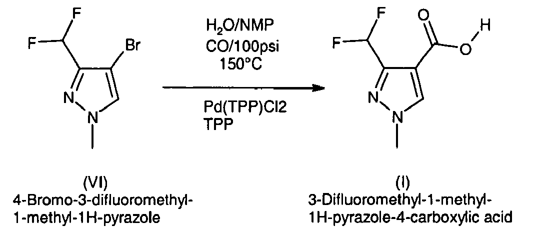

0.31 g 4-Bromo-3-difluoromethyl-1-methyl-1 H-pyrazole, 0.82 g water, 9 g N-methylpyrrolidinone, 1 g triethylamine, 0.06 g tris(triphenylphosphonium)palladium(II)chloride and 0.12 g triphenyl phosphine were charged to a 50ml glass miniclave reactor fitted with a 10bar bursting disc, thermometer and gas feed. The system was pre-purged with carbon monoxide six times up to 7 bar - venting off slowly each time and agitating throughout. Finally the reactor was pressurised up to 6 bar. The system was agitated whilst heating up to 150°C and the pressure maintained at 6-7 bar by venting as necessary. When the mixture had stabilised on temperature, and pressure, it was agitated overnight under these conditions. The following day the system was cooled, vented and the reactor contents evaporated as far as possible on a rotary evaporator. The resultant oil was quenched with an aqueous base and extracted with 3 x 25ml of ether. The combined ether extracts were washed with brine, dried over magnesium sulphate and concentrated under vacuo to yield a red/brown oil (0.5g). The aqueous base extracts were acidified and back extracted with 3 x 25ml of ether. The extracts were combined, dried over magnesium sulphate and concentrated under vacuo. Derivatised GC analysis showed that the desired 1, 3-pyrazole isomer (compound of formula I) was present in virtually all phases obtained from the work-up (HPLC analysis). Most of the product, and its isomer, were present in the ether extract from the initial base wash. The product was not isolated but compared to reference material by HPLC, derivatised GC and GCMS.

### Preparation of (I) from (XIII) via (XII), (XI) and (II):

### Example C2: Preparation of (XI) from (XIII) via (XII):

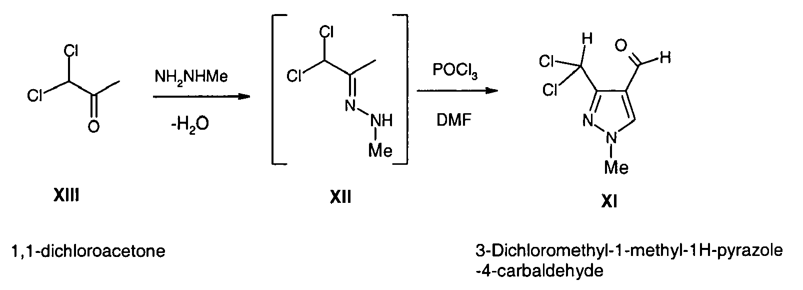

A 50ml round bottomed flask was fitted with a magnetic stirrer, thermometer and nitrogen atmosphere. Dimethylformamide (30ml) and 1,1-dichloroacetone (compound of formula XIII) (3.05g) were charged to the reactor. Methylhydrazine (1.25g) was added slowly to the agitated solution, maintaining the mixture below 25°C. The reaction was stirred at ambient temperature for 0.75h to give the desired hydrazone intermediate (compound of formula XII). A 150ml round bottomed flask was fitted with a magnetic stirrer, condenser, thermometer and nitrogen atmosphere. Dimethylformamide (60ml) was charged to the reactor and heated to 50°C. Phosphorous oxychloride (15.0g) was added via syringe pump. The reaction was stirred at 50°C for 1 h then cooled to 10°C. The hydrazone (compound of formula XII) solution prepared as described above was charged immediately, maintaining the temperature at 5-10°C. The reaction was stirred at 80°C for 5h and then cooled to ambient temperature. The reaction mass was divided into two equal parts. Dichloromethane (100ml), water (100ml) and 10% aqueous sodium bicarbonate solution (150ml) were charged to a glass vessel. The first half of the reaction mass was added and the pH adjusted to 7-8 with further sodium bicarbonate solution. This procedure was repeated with the second half of the reaction mass. The combined organic layers were washed with water (2 x 100ml), dried (MgSO₄) and concentrated *in vacuo.* The compound of formula XI was analyzed by MS and NMR.
MS: 42, 50, 85, 94, 122, 157, 192 (M⁺)
¹H NMR (D7-DMF): 3.97 (s, 3H, NCH₃), 7.20 (s, 1H, CHCl₂), 8.05 (S, 1H, ArH), 10.03 (s, 1H, CHO)

### Example C3: Preparation of (II) from (XI):

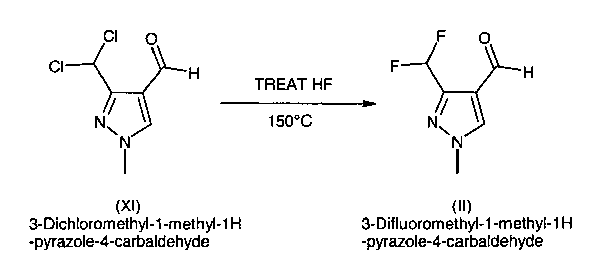

3-Dichloromethyl-1-methyl-1 H-pyrazole-4-carbaldehyde (1.94g, compound of formula XI) was charged to a Monel 100ml pressure reactor followed by 20.1 g tris(hydrogen fluoride)-triethylamide by syringe. The system was sealed up and agitated whilst heating the contents to 150°C. After achieving the target temperature the reaction mass was held on temperature for 2h. The reaction mass was then allowed to stand overnight and cool before it was quenched. Quenching was effected by drowning out the reactor contents (black liquid) into water (50ml). The quenched reaction mass was then extracted with methyl-t-butyl ether (2 x 25ml). After separating the organic phases were washed with brine and the organic layer was dried with magnesium sulphate, filtered and concentrated under vacuo to give the compound of formula II in the form of a red/brown oil (0.38g); yield ~40%. The product was analysed by GC and GCMS.
GCMS: 42, 51, 69, 77, 83, 112, 131, 141, 159, 160 (M⁺)

The compound of formula (I) can be formed from the compound of formula (II) via step a3) as described above.

### Preparation of (I) from (XV) via (XVI), (XIV) and (V):

### Example C4: Preparation of (XIV) from (XV) via (XVI):

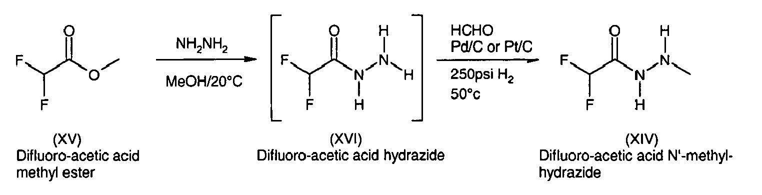

Hydrazine hydrate (4.62g) was charged to a 3-necked round bottomed flask. The flask was fitted with a thermometer, condenser and syringe feed. The system was back pressured on the condenser outlet with a nitrogen purge/bubbler. Methanol (75ml) was charged to the reactor and the mixture agitated under nitrogen. Methyldifluoroacetate (1 0.2g, compound of formula XV) was dissolved in methanol (17ml) and fed, by syringe, into the reactor over 75 minutes. The resultant mixture was transferred to a 300ml Hastelloy Parr reactor.

Formaldehyde (7.4g) was charged to the reactor and the mixture agitated for 30 minutes at room temperature accompanied by a small exotherm reaction. 1 g Pt/C was added and washed in with methanol (20ml). The Parr reactor was sealed, purged with nitrogen three times, purged with hydrogen three times (all to 200psi) and then pressurised up to 200psi with hydrogen. Agitation and external heating were applied to heat the reaction to 50°C. The reactor was held under these conditions for 3h and then allowed to cool. The system was purged three times with nitrogen to 200psi. The contents were removed and filtered. The solvent was removed under vacuum, filtered and concentrated under vacuo. The compound of formula XIV was obtained in the form of an orange oil (9.1g). Yield 40% total isomers and 36% of the desired isomer. The product was analysed by GC, GCMS and NMR (¹H and ¹⁹F).
GCMS: 45, 51, 60, 75, 79, 96, 124 (M⁺)
For the desired product: ¹H NMR (CDCl₃): 2.67 (s, 3H, CH₃N), 5.95 (t, 1 H, CHF₂);
¹⁹F NMR (CDCl₃): -127.92 (d, CHF₂)
For the isomer: ¹H NMR (CDCl₃): 2.59 (s, 3H, CH₃N), 5.92 (t, 1 H, CHF₂); ¹⁹F NMR (CDCl₃): - 127.94 (d, CHF₂)

### Example C5: Preparation of (V) from (XIV):

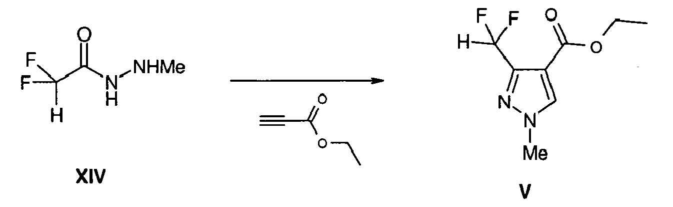

A 25ml reaction tube was fitted with a magnetic stirrer, thermometer, condenser and nitrogen atmosphere. Dimethylformamide (10g), ethyl propargylate (0.14g), the compound of formula XIV (0.24g) and p-toluene sulphonic acid (0.028g) were charged to the reaction tube. The mixture was stirred at 50°C overnight, 80°C overnight and then 110°C overnight to give the desired product (compound of formula V).
MS: 42, 43, 112, 132, 140, 159, 176, 204 (M⁺)
¹H NMR (CDCl₃): 1.35 (t, 3H, CH₃CH₂), 3.97 (s, 3H, NCH₃), 4.32 (q, 2H, CH₃CH₂O), 7.25 (t, 1 H, CHF₂), 7.90 (S, 1 H, ArH)

The compound of formula (I) can be formed from the compound of formula (V) via step b6) as described above.

### Preparation of (I) from (XV) via (XIV) and (V):

### Example C6: Direct preparation of (XIV) from (XV):

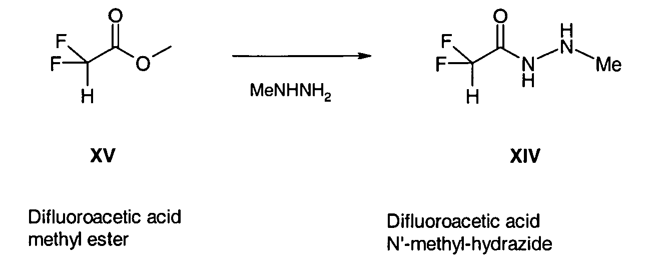

A 3-neck 250ml round bottomed flask was fitted with a magnetic stirrer, thermometer and nitrogen atmosphere. Tetrahydrofuran (120ml), methyl difluoroacetate (compound of formula XV; 4g) and methylhydrazine (1.65g) were charged, and the mixture stirred at ambient temperature for 4h. Further methyl hydrazine (0.38g) was added and the mixture stirred for a further 3h at ambient temperature. The desired product (compound of formula XIV) was formed as a 3:1 mixture with its N-regioisomer and was isolated by concentration *in vacuo.*
MS: 45, 51, 60, 75, 79, 96, 124 (M⁺)
¹H NMR (CDCl₃): 2.67 (s, 3H, CH₃N), 5.95 (t, 1H, CHF₂)

The compound of formula (V) can be formed from the compound of formula (XIV) via the methology described in comparative example C5 above. The compound of formula (I) can be formed from the compound of formula (V) via step b6) as described above.

### Preparation of (I) from (XIX) via (XVIII), (XVII) and (V):

### Example C7: Preparation of (XVIII from (XIX):

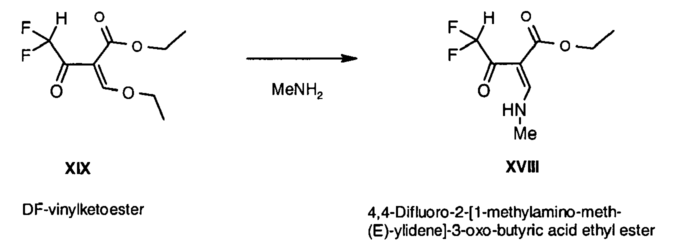

A 3-neck 50ml round bottomed flask was fitted with a magnetic stirrer, thermometer, and nitrogen atmosphere. Dichloromethane (36ml) and the compound of formula XIX (5.0g) were charged to the reactor and the resulting solution cooled to 0°C. Methylamine (4.20g) was added over 0.25h, maintaining the temperature below 5°C, and the reaction stirred at ambient temperature for 1.5h. Concentration *in vacuo* gave a yellow solid, which was washed with isohexane (60ml, 20ml). The compound of formula XVIII was obtained as an off-white solid (86% yield).
MS: 42,43, 55, 84, 112, 128, 156, 162, 207 (M⁺)
¹HNMR(CDCl₃): 1.32 (t, 3H, CH₃CH₂), 3.25 (d, 3H, NHCH₃), 4.23 (q, 2H, CH₃CH₂O), 6.88 (t, 1H, CHF₂), 8.10 (d, 1H, C=CH), 10.83 (br s, 1H, =CH-NH-CH₃)

### Example C8: Preparation of (V) from (XVIII) via XVII):

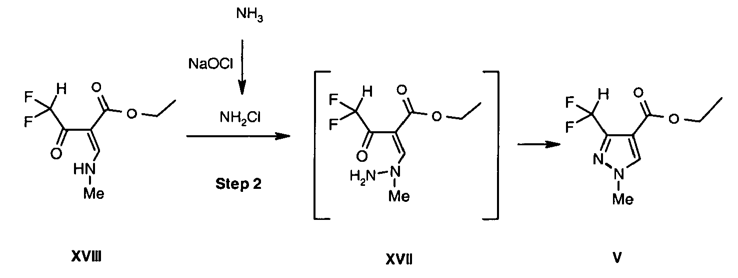

### a) Preparation of chloramine:

A 100ml conical flask was fitted with a magnetic stirrer and thermometer. Diethyl ether (31 ml) and ammonium chloride (0.91 g) were charged to the flask. The mixture was cooled to -10°C and aqueous ammonia solution (1.7g) was added. Sodium hypochlorite (14.3g) solution was charged over 10 minutes to the vigorously stirred solution at -10°C, and the reaction was then stirred at -10°C for 0.5hr. The organic layer was separated, washed with brine, dried over CaCl₂ for 1 hr at -15°C. The desired chloramine was generated as a 3% chloramine solution in diethylether.

### b) Preparation of the compound of formula V via the compound of formula XVII:

A 3-neck round bottomed flask was fitted with a magnetic stirrer, thermometer and nitrogen atmosphere. Sodium hydride in mineral oil (0.28g, 6.82mmol, 5.5eq) was charged to the reactor. The obtained paste was triturated with isohexane (2 x 10ml) to remove the mineral oil, then tetrahydrofuran (25ml) and 0.26 g of the compound of formula XVIII (prepared as described in comparative example 7) were added. The mixture was stirred at ambient temperature for 0.5h to give a clear solution. This tetrahydrofuran solution was added slowly to the chloramine/diethylether-solution described above (6.22mmol, 5.0eq) at -15°C. The reaction was stirred at -15°C for 1 h and then allowed to warm to ambient temperature. A new solution of chloramine in diethylether (2.7% strength) was prepared as described above. Additional sodium hydride dispersion (0.20g, 4.96mmol, 4.0eq) and chloramine (new solution) (10.4g, 5.46mmol, 4.4eq) were added and the reaction was stirred for 1 h at ambient temperature. A final charge of sodium hydride dispersion (0.22g, 5.46mmol, 4.4eq) and chloramine (1 0.7g, 5.58mmol, 4.5eq) was added to the reactor. The reaction was stirred at ambient temperature overnight to give the desired compound of formula V.
MS: 42, 43, 112, 132, 140, 159, 176, 204 (M⁺)
¹H NMR (CDCl₃): 1.35 (t, 3H, CH₃CH₂), 3.97 (s, 3H, NCH₃), 4.32 (q, 2H, CH₃CH₂O), 7.25 (t, 1 H, CHF₂), 7.90 (S, 1 H, ArH)

The compound of formula (I) can be formed from the compound of formula (V) via step b6) as described above.

## Claims

1. A process for the production of a compound of formula I 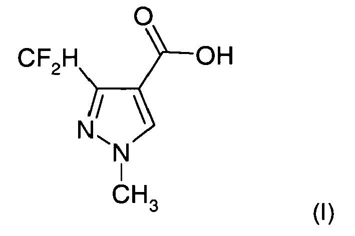 which comprises
a1) reacting a compound of formula IV (1,1-difluoroacetone) 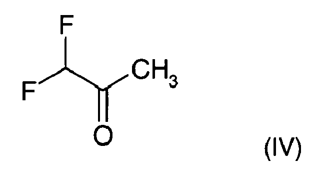 with methylhydrazine to form a compound of formula III 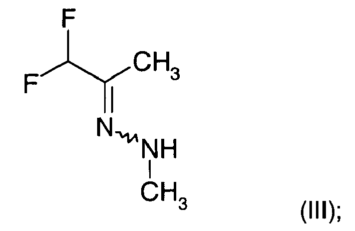
a2) converting that compound with dimethyl formamide and an activating agent into a compound of formula II 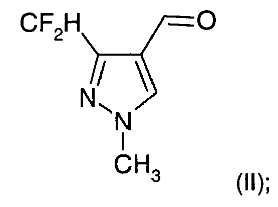 and
a3) oxidizing that compound with an oxidizing agent in the presence of a base to the compound of formula (I).

2. Compound of formula II

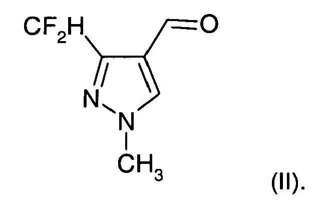

3. Compound of formula III

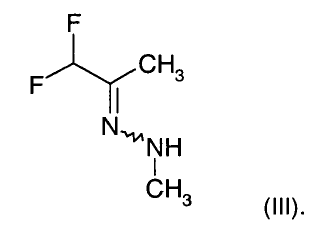

4. A process for the production of a compound of formula I 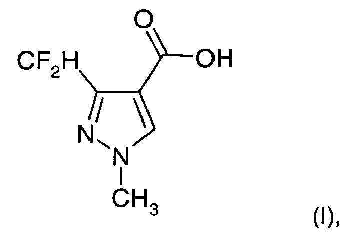 which comprises
b1) reacting a compound of formula X (dichloroacetyl chloride) 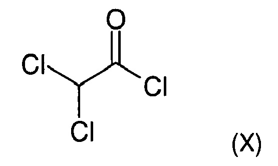 with a compound of formula Xb 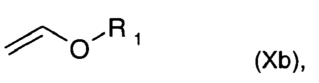 wherein R₁ is C₁-C₆alkyl, to form a compound of formula IX 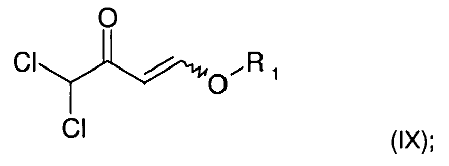 wherein R₁ is as defined under formula Xb;
b2) converting that compound with methylhydrazine into a compound of formula VIII (3-dichloromethyl-1-methyl-1 H-pyrazole) 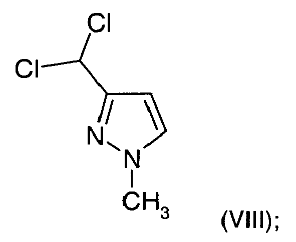
b3) fluorinating that compound with a fluorinating agent into a compound of formula VII (3-difluoromethyl-1-methyl-1 H-pyrazole) 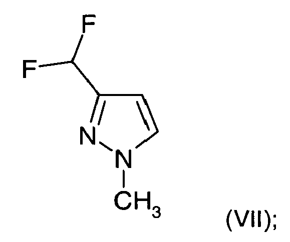
b4) brominating that compound with a brominating agent into a compound of formula VI (4-bromo-3-difluoromethyl-1-methyl-1 H-pyrazole) 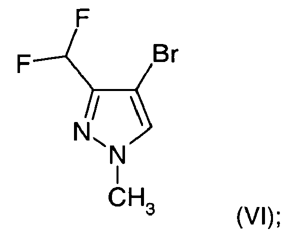
b5) converting that compound with carbon monoxide and a C₁-C₆alkanol in the presence of a palladium catalyst into a compound of formula V 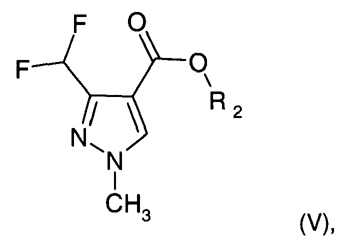 wherein R₂ is C₁-C₆alkyl; and
b6) saponifying that compound leading to the formation of the compound of formula (I) by b6.1) adding a hydroxide base or water in the presence of a base to form the anion of the compound of formula (I) and then adding an acid to form the compound of formula (I); or b6.2) adding water in the presence of an acid to form the compound of formula (I).

5. Compound of formula VI

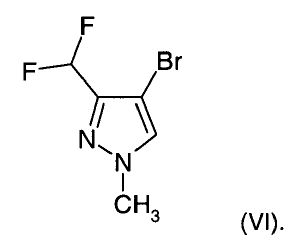

6. Compound of formula VII

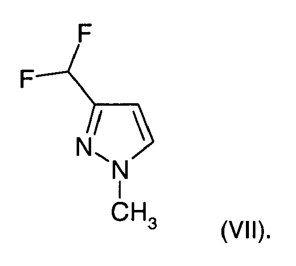

7. Compound of formula VIII
